# EUROPEAN PATENT APPLICATION

(11) **EP 4 575 498 A1**
(43) Date of publication of application: **25.06.2025**
(21) Application number: 23219733.5
(22) Date of filing: 22.12.2023
(51) Int. Cl.: G01N 33/52, B32B 37/14

(54) **SUSTAINABLE BACKING FOR LATERAL FLOW IMMUNOASSAY AND DIAGNOSTIC PURPOSE**

(71) Applicant: Ahlstrom Oyj, 02150 Espoo (FI)
(72) Inventor: BEBIEN, Frédéric, 38200 Jardin (FR); CONSIGLIO, Marco, 10149 Torino (IT)
(74) Representative: Laine IP Oy

(57) **Abstract**

According to an example aspect of the present invention, there is provided a sustainable rapid test strip and/or kit comprising a cellulose-based backing support of certain density.

## Description

### FIELD

The present technology relates to rapid test strips and/or kits utilizing sustainable, renewable, biodegradable and/or compostable materials.

### BACKGROUND

Lateral Flow Immunochromatographic Assay (LFIA) is an affordable technology aiming at rapid detection of an analyte within a short period of time. The target analytes of lateral flow assays include infectious disease agents, drugs of abuse, hormones, cancer markers, food contaminants, agricultural contaminants and such. In the common and known LFIA-tests, there is a strip supporting pieces of fibre-based and membrane materials, i.e. backing supports, which holds the reactive part of the test. Sukumaran A., *et al.* (2021) gives a good basic understanding about the principles, development and problems of LFIAs.

Most backings used in test strips and kits currently on the market are made from oil-based plastic materials, such as polyvinyl chloride (PVC), polyethylene terephthalate (PET) or polystyrene (PS). They provide good dimensional stability and are easily cut into very narrow pieces. However, such backings are not from sustainable sources and are not compostable and/or biodegradable.

In addition, some of these plastic components contain fluorophore material such as optical brightener or fluorescent chemical compounds, which interferes with the reading of the results, in particular, for quantitative tests using fluorescent beads.

Some attempts have been made to replace the use of plastic materials as a backing support.

For example, JP 2847532 B2 discloses the application of carboxymethyl cellulose or carboxyethyl cellulose as the backing support for bodily fluid inspecting kit. However, such backings are not sustainable in terms of compostability and/or biodegradability.

Another example, US 2002/071784 A1, discloses a dry analytical kit that uses transparent chemically modified cellulose as its backing support. It comprises a polyester layer coated with an organic solvent and requires use of non-sustainable chemicals.

There is thus a need for novel rapid test strips and/or kits, wherein the backing is based on a sustainable material and yet has at least the same features and mechanical properties of for example stiffness, thickness and/or ability to accept PSAs than the corresponding state of the art plastic-based backing.

### SUMMARY OF THE INVENTION

The invention is defined by the features of the independent claims. Some specific embodiments are defined in the dependent claims.

According to an aspect of the present invention, there is provided a rapid test strip and/or kit that includes a backing support, which comprises cellulose and/or modified cellulose material(s) and has a certain density.

This and other aspects, together with the advantages thereof over known solutions are achieved by the present invention, as hereinafter described and claimed.

The test strip and/or kit of the present invention is mainly characterized by what is stated in the characterizing part of claim 1.

Considerable advantages are obtained by means of the invention. For example, the present solution is able to replace the current plastic-based backings on the market. The backing of the present invention is also able to address the accuracy of density and/or thickness, which is one key factor in lateral flow immunoassays. In addition, the material of the present invention is made from renewable resources, is biodegradable and/or industrially compostable according to EN 13432. Furthermore, the present invention enables reading of results via conventional visual readers and via quantification of fluorescent beads. Another advantage of the present invention is that it can be produced by using a paper manufacturing process.

Next, the present technology is described more closely with reference to certain embodiments.

### EMBODIMENTS

The present technology provides a novel cellulose based backing support material used in rapid test strips and/or kits for lateral flow immunoassays and diagnostics purposes.

Abbreviation "LFIA" herein means lateral flow immunoassay, lateral flow immunochromatographic assay and such.
FIGURE 1 is a drawing showing an exploded view of a typical lateral flow strip test (1) and the different components thereof. The strip comprises different overlapping components in sequential order, a sample pad (2), a conjugate pad (3), a nitrocellulose membrane (4) with a test line (41) and a control line (42), and an absorbent pad (5). The different components are arranged on a backing support (6).
FIGURE 2a is a drawing of a single layered backing (6).
FIGURE 2b is a drawing of an assembly of a multi-layered backing support (6) according to one embodiment, comprising a top layer (61) with dense paper, an adhesive (62) and a bottom layer (63) to add bulk (thickness).
FIGURE 3 is a photo showing sustainable backing supports according to one embodiment, cut down in strips of 5 mm wide and 60 mm long, as currently made for standard test strips formats.

One aspect of the present invention is a stiff medium of high density with precise thickness able to accept for example a pressure sensitive adhesive and a release liner as those used currently in diagnostic applications. Such medium is used as a backing to hold the reactive part of a rapid test strip and/or kit.

Thus, one embodiment is a rapid test strip and/or kit comprising a backing support 6, wherein the backing support 6 comprises cellulose and/or modified cellulose material(s) and has a has a density of at least 0.75 g/m³, preferably between 0.78 and 1.20 g/m³. The density is measured by first measuring the thickness according to ISO 9073-2-1995 and the basis weight according to ISO 536-2019, and then by dividing the basis weight by the thickness.

According to one embodiment, the cellulose and/or modified cellulose material(s) comprised in the backing support 6 are cellulose fibres and/or modified cellulose fibres.

A further embodiment is a rapid test strip and/or kit comprising a backing support 6, wherein the backing support 6 has a thickness between 200 and 400 µm, preferably between 250 and 380 µm. These thicknesses match with the market demand for current PVC and PS materials. The thickness is determined according to ISO 9073-2-1995.

According to one embodiment of the present invention, at least one surface of the backing support 6 has a surface energy of at least 250 mN/m. This feature for example enables acceptance of an adhesive 62 on top of the backing support 6 with good adhesion. The surface energy is measured for example by using a Digidrop apparatus manufactured by GBX instrument.

According to one embodiment of the present invention, at least one surface of the backing support 6 has a Cobb value less than 30 g/m², preferably less than 25 g/m². This feature for example limits absorption of an adhesive during the lamination/assembly process of the test kit. This is also advantageous when the backing support 6 is used in a dipstick format for maintaining structural integrity during use. The Cobb value is measured according to ISO 535:2014.

According to one embodiment of the present invention, the backing support 6 has brightness of whiteness index of at least 25, more preferably about 50, as determined under CIE D65/10° according to ISO 11475:2000. This feature for example enables the LFIA to provide high level of performance of the test in the final application. Furthermore, the diagnostic market is currently not ready for switching to unbleached supports, because of high purity requirements.

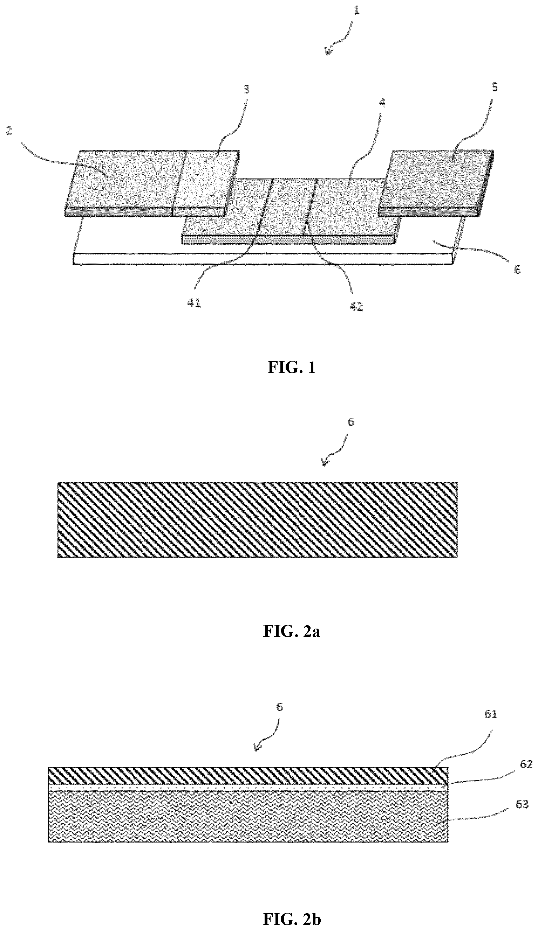

According to one embodiment of the present invention, the backing support 6 is single layered, as shown on Figure 2a.

According to another embodiment of the present invention, the backing support 6 is multi-layered. An example of a multi-layered backing support is shown on Figure 2b. It is preferred that the layers are assembled with an adhesive 62 of less than 8 g/m², which is required for achieving the thickness targets. Suitable adhesives include for example acrylic adhesives, latex, resins, polylactic acid (PLA) dispersions, hot-melt adhesives, or natural adhesives such as starch.

According to a further embodiment of the present invention, the layers are assembled with an adhesive 62 representing less than 20 wt-%, preferably less than 10 wt-%, and more preferably less than 5 wt-% of the total weight of the backing support 6.

According to one embodiment of the present invention, the backing support 6 comprises at least one dense paper layer 61, selected from vegetable parchment, glassine, tracing paper, impregnated paper, coated paper, paper made with refined pulp, calendered paper and supercalendered paper. In the context of the present invention, a dense paper is a paper having density of at least 0.75 g/m³. The density is measured similarly as defined earlier.

According to one embodiment of the present invention, the backing support 6 is renewable, biodegradable and/or industrially compostable material, as defined by EN 13432. This is advantageous because it reduces the use of plastics, which are non-renewable and/or non-recyclable.

According to one embodiment of the present invention, the backing support 6 is substantially fluorophore free. In the context of the present invention, a backing support substantially fluorophore free should be understood as a backing support having a difference between whiteness under CIE UV D65/10° and whiteness under CIE D65/10° of less than 0.5, when measured according to ISO 11475:2000. Known plastic based backing supports may contain fluorophore, which is not suitable for quantitative rapid tests requiring reading of the results of fluorescent beads. This embodiment therefore provides a solution that does not interfere with the results obtained via a fluorescence reader.

According to one embodiment of the present invention, the backing support 6 has a dimensional stability of 2 in a scale of 1 to 5, where 1 is low deformation and 5 is high deformation, at 38°C 90% relative humidity after 48h.

According to one embodiment of the present invention, the backing support 6 has a hydrophobic surface treatment on at least one of its surfaces. In particular, for a dipstick application the absorption of the liquid sample to be analyzed should be avoided. Having a hydrophobic treatment prevents absorption.

According to one embodiment of the present invention, the backing support 6 has cross direction (CD) stiffness of at least 1100 mN, preferably at least 1700 mN, and Machine direction (MD) stiffness of at least 2300 mN, preferably at least 3500 mN. The stiffness is measured according to ISO 2493-2 for example on a Lorentzen & Wettre apparatus.

Use of the test strip and/or kit according to the present invention in a dipstick or cassette format also belongs to the scope of the present invention.

Reference throughout this specification to one embodiment or an embodiment means that a particular feature, structure, or characteristic described in connection with the embodiment is included in at least one embodiment of the present invention. Thus, appearances of the phrases "in one embodiment" or "in an embodiment" in various places throughout this specification are not necessarily all referring to the same embodiment. Where reference is made to a numerical value using a term such as, for example, about or substantially, the exact numerical value is also disclosed.

As used herein, a plurality of items, structural elements, compositional elements, and/or materials may be presented in a common list for convenience. However, these lists should be construed as though each member of the list is individually identified as a separate and unique member. While the forgoing examples are illustrative of the principles of the present invention in one or more particular applications, it will be apparent to those of ordinary skill in the art that numerous modifications in form, usage and details of implementation can be made without the exercise of inventive faculty, and without departing from the principles and concepts of the invention. Accordingly, it is not intended that the invention be limited, except as by the claims set forth below.

The verbs "to comprise" and "to include" are used in this document as open limitations that neither exclude nor require the existence of also un-recited features. The features recited in depending claims are mutually freely combinable unless otherwise explicitly stated. Furthermore, it is to be understood that the use of "a" or "an", that is, a singular form, throughout this document does not exclude a plurality.

### INDUSTRIAL APPLICABILITY

The solution of the present invention is industrially applicable for example for providing rapid tests for lateral flow immunoassays and diagnostics purposes, such as for testing of infectious diseases, agricultural samples, food and allergen samples, animal screening, drug samples, human body fluid samples and such.

### EXAMPLES

At lab scale, the surface of a dense paper was coated with a water-based adhesive with Tg = 5°C using a lab hand coater and Smooth bar. Then the treated paper was dried for 2 min at 90°C. Then another ply of the same dense paper or another one was laid down over the precoated material. It was finally pressed and dried with glazing plate for 1 min at 120°C. From this process, the sustainable backing support was made.

The physical properties of certain embodiments are shown in Tables 1 and 2.

**Table 1: Physical properties of starting materials**

| | Sample 1.1 | Sample 1.2 | Sample 1.3 | Sample 1.4 | Sample 1.5 |
|---|---|---|---|---|---|
| Edana Thickness 1,96KPa (µm) | 96 | 235 | 275 | 109 | 144 |
| CV EDANA 1,96KPa Thickness (%) | 1,8 | 1,4 | 1,4 | 1,2 | 3,2 |
| Density EDANA (g/cm³) | 0,75 | 0,79 | 0,85 | 1,1 | 1 |
| Water Cobb 60 (g/m²) | 36 | 22 | 20 | 22 | 22 |

Different samples were used to prepare a backing support.

Sample 1.1: is a vegetable parchment with a 70 g/m² basis weight.

Sample 1.2: is a cellulosic paper made using 20% of short fibers and 80% of long fibers. The fibers are refined at 30-40°SR. The paper has a basis weight of 185 g/m².

Sample 1.3: is a cellulosic paper made 100% of long fibers. The fibers are refined at 30-40°SR. The paper has a basis weight of 230 g/m².

Sample 1.4 is a supercalendered glassine paper with 14 wt% of short fibers and 86% if long fibers. The fibers are refined at 60-70 °SR.

Sample 1.5 is a supercalendered glassine paper with 30 wt% of short fibers and 59 wt% and 12 wt% of CTMP fibers.

The different samples were laminated or used as such to prepare the backing support according to the invention. The different samples were then cut in a 5 mm by 60 mm strips to measure the dimensional stability.

Sample 2.1 was prepared by laminating sample 1.1 and a porous cellulosic substrate with a density of 0.38 g/m³ and a thickness of 230 µm.

Sample 2.2 was obtained after calendering of sample 1.2.

Sample 2.3 was obtained after calendering sample 1.3.

Sample 2.4 was obtained by coating sample 1.4 with a water-based adhesive with Tg = 5°C using a lab handcoater and Smooth bar. Then the treated paper was dried for 2min at 90°C. Another ply of sample 1.4 was laid down over the pre-coated sample 1.4. The assembly was pressed and dried with glazing plate for 1 min at 120°C.

Sample 2.5 was obtained by coating sample 1.4 with a water-based adhesive with Tg = 5°C using a lab hand coater and smooth bar. Then the treated paper was dried for 2 min at 90°C. Another ply of sample 1.5 lay down over the pre-coated sample 1.5. The assembly was pressed and dried with glazing plate for 1 min at 120°C.

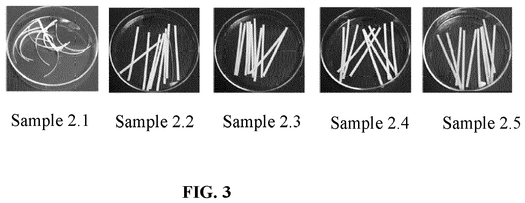

In order, to test the dimensional stability of the different samples, they are 90% relative humidity at 38°C and aged for 48H. After visual inspection the dimensional stability is scored as follows 1 for low deformation and 5 for high deformation. The different samples are shown on figure 3.

**Table 2: Physical properties of different backing supports**

| | Sample 2.1 | Sample 2.2 | Sample 2.3 | Sample 2.4 | Sample 2.5 |
|---|---|---|---|---|---|
| EDANA Thickness 1,96KPa (µm) | 316 | 235 | 275 | 207 | 262 |
| CV Thickness (%) | 3,9 | 1,4 | 1,4 | 2,8 | 1,4 |
| Density (g/cm3) | 0,53 | 0,79 | 0,85 | 1,17 | 1,09 |
| Lorentzen Stiffness MD (mN) | 763 | 1790 | 2432 | 1866 | 2687 |
| Lorentzen Stiffness CD (mN) | 1257 | 3515 | 5277 | 2483 | 4095 |
| Peel Adhesion on N/inch - 24H@RT] | 25,5 | 23,8 | 24,4 | 26,0 | 25,7 |
| Total Thickness Double side w/Relelease liner (µm) | 528 | 451 | 494 | 427 | 483 |
| Whiteness CIE D65/10° | 72,84 | 26,09 | 16,68 | 66,14 | -84,37 |
| Whiteness CIE-UV D65/10 | 72,61 | 25,8 | 16,35 | 65,95 | -84,3 |
| Dimensional stability 38°C 90%RH 48H (1-5, 1 low deformation 5 high deformation) | 5 | 2 | 2 | 2 | 2 |

Given that all samples 1.1 to 1.5 are produced on a paper machine, fibers are more oriented in the machine direction. Consequently, said samples will have different physical properties such as stiffness in the machine direction (MD) and the cross direction (CD).

Furthermore, inventors observed that samples having a higher Cobb value tend to curl more. Without wishing to be bound by theory, a sample with a higher Cobb tends to absorb more adhesive used for lamination and create structure asymmetry within the sample. When exposed to moisture the asymmetry will cause one surface to expand more than the other, thus curl more.

As seen from the examples, the products disclosed are performing well in terms of dimensional stability, which is an important factor because curling can deform the different components that are going to assembled on the backing card, thus affecting the analysis. The high density and the high stiffness of the samples according to the invention is important notably for maintaining good dimensional stability.

### CITATION LIST

### Patent literature

1. JP 2847532 B2
2. US 2002/071784 A1

### Non-patent literature

1. Sukumaran A., Thomas T., Thomas R., Thomas R. E., Paul J. K., Vasudevan D. M., Development and Troubleshooting in Lateral Flom Immunochromatography Assays, Indian J Clin Biochem. 2021 Apr; 36(2): 208-212, doi: 10.1007/s12291-020-00887-5.

## Claims

1. A rapid test strip and/or kit comprising a backing support (6), **characterized in that** the backing support (6) comprises cellulose and/or modified cellulose material(s) and has a density of at least 0.75 g/m³, preferably between 0.78 and 1.20 g/m³.

2. The rapid test strip and/or kit according to claim 1, **characterized in that** the cellulose and/or modified cellulose material(s) comprised in the backing support (6) are cellulose fibres and/or modified cellulose fibres.

3. The rapid test strip and/or kit according to claim 1 or 2, **characterized in that** the backing support (6) has a thickness between 200 and 400 µm, preferably between 250 and 380 µm.

4. The rapid test strip and/or kit according to any of the preceding claims, **characterized in that** at least one surface of the backing support (6) has a surface energy of at least 250 mN/m.

5. The rapid test strip and/or kit according to any of the preceding claims, **characterized in that** at least one surface of the backing support (6) has a Cobb value less than 30 g/m², preferably less than 25 g/m².

6. The rapid test strip and/or kit according to any of the preceding claims, **characterized in that** the backing support (6) has a brightness of whiteness index of at least 25, more preferably at least 50.

7. The rapid test strip and/or kit according to any of the preceding claims, **characterized in that** the backing support (6) is multi-layered.

8. The rapid test strip and/or kit according to claim 7, **characterized in that** the layers are assembled with an adhesive (62) representing less than 20 wt-%, preferably less than 10 wt-%, and more preferably less than 5 wt-% of the total weight of the backing support (6).

9. The rapid test strip and/or kit according to any of the preceding claims, **characterized in that** the backing support (6) comprises at least one dense paper layer (61), selected from vegetable parchment, glassine, tracing paper, impregnated paper, coated paper, paper made with refined pulp, calendered paper and supercalendered paper.

10. The rapid test strip and/or kit according to any of the preceding claims, **characterized in that** the backing support (6) is biodegradable and/or industrially compostable.

11. The rapid test strip and/or kit according to any of the preceding claims, **characterized in that** the backing support (6) is substantially fluorophore free.

12. The rapid test strip and/or kit according to any of the preceding claims, **characterized in that** the backing support (6) has a hydrophobic surface treatment on at least one of its surfaces.

13. The rapid test strip and/or kit according to any of the preceding claims, **characterized in that** the backing support (6) has cross direction (CD) stiffness of at least 1100 mN, preferably at least 1700 mN, and/or machine direction (MD) stiffness of at least 2300 mN, preferably at least 3500 mN.

14. Use of the rapid test strip and/or kit according to any of the preceding claims in a dipstick or cassette format.

15. The use according to claim 14 for testing of infectious diseases, agricultural samples, food and allergen samples, animal screening, drug samples and human body fluid samples.
